# EUROPEAN PATENT SPECIFICATION

(11) **EP 0 589 961 B1**
(45) Date of publication and mention of the grant of the patent: **20.03.2002**
(21) Application number: 92912036.8
(22) Date of filing: 23.04.1992
(51) Int. Cl.: C12N 15/12, C07K 14/00, C12N 5/10, G01N 33/68, A01K 67/027, A61K 38/00

(54) **ASSAY SYSTEMS FOR NEUROTROPHIN ACTIVITY**
NACHWEISSYSTEM FÜR DIE NEUROTROPHIN-AKTIVITÄT
SYSTEMES D'ANALYSE PERMETTANT DE DETECTER L'ACTIVITE DE LA NEUROTROPHINE

(30) Priority: 23.04.1991 US 690199; 26.07.1991 US 736559
(43) Date of publication of application: 06.04.1994
(60) Divisional application: 99203310.0
(73) Proprietor: REGENERON PHARMACEUTICALS, INC., Tarrytown, NY 10591-6707 (US)
(72) Inventor: SQUINTO, Stephen P., Connecticut 06524 (US); GLASS, David, New York, NY 10032 (US); ALDRICH, Thomas, H., New York, NY 10021 (US); DiSTEPHANO, Peter, Carmel, NY 10512 (US); STITT, Trevor, Huntington Station, NY 11746 (US); FURTH, Mark, E., Pelham, NY 10803 (US); YANCOPOULOS, George, D., Briarcliff Manor, NY 10510 (US); MAISONPIERRE, Peter, C., Croton, NY 10520 (US); MASIAKOWSKI, Piotr, Milford, CT 06460 (US); IP, Nancy, Stamford, CT 06902 (US)
(74) Representative: Goldin, Douglas Michael
(86) International application number: US9203376
(87) International publication number: WO9218149

(56) References cited:
- EP-A- 0 504 914
- WO-A-92/16559
- US-A- 4 963 481
- NATURE. vol. 350 , 14 March 1991 , LONDON GB pages 158 - 160 KAPLAN DR;MARTIN-ZANCA D;PARADA LF; 'Tyrosine phosphorylation and tyrosine kinase activity of the trk proto-oncogene product induced by NGF.'
- EMBO Journal, Volume 8, No. 12, issued 1989, R. KLEIN et al., "TrkB, a novel tyrosine protein kinase receptor expressed during mouse neural development", pages 3701-3709, see entire document.
- Development, Volume 109, issued 1990, R. KLEIN et al ., "Expression of the tyrosine kinase receptor gene trkB is confined to the murine embryonic and adult nervous system", pages 845-850.
- Cell, Volume 61, issued 18 May 1990, R. KLEIN et al., "The trkB Tyrosine Protein Kinase Gene Codes for a Second Neurogenic Receptor that Lacks the Catalytic Kinase Domain", pages 647-656, see entire document.
- Cell, Volume 65, issued 05 April 1991, R. KLEIN et al., "The trk Proto-Oncogene Encodes a Receptor for Nerve Growth Factor", pages 189-197, see entire document.
- Molecular and Cellular Biology, Volume 6, No. 10, issued October 1986, X. ZHAN et al., "Growth Factor Requirements of Oncogene-Transformed NIH 3T3 and BALB/c 3T3 Cells Cultured in Defined Media", see entire document.
- Molecular and Cellular Biology, Volume 9, No. 1, issued January 1989, D. MARTIN-ZANCA et al., "Molecular and Biochemical Characterization of the Human trk Proto-Oncogene", pages 24-33, see entire document.
- Cell, Volume 66, No. 2, issued 26 July 1991, D.J. GLASS et al., "TrkB Mediated BDNF/NT-3-Dependent Survival and Proliferation in Fibroblasts Lacking the Low Affinity NGF Receptor", pages 405-413, see entire document.
- Cell, Volume 65, No. 5, issued 31 May 1991, S.P. SQUINTO et al., "trkB Encodes a Functional Receptor for Brain-derived Neurotrophic Factor and Neurotrophin-3 but Not Nerve Growth Factor", pages 885-893, see entire document.
- Cell, Volume 65, No. 5, issued 31 May 1991, D. SOPPET et al., "The Neurotrophic Factors Brain-Derived Neurotrophic Factor and Neurotrophin-3 are Ligands for the trkB Tyrosine Kinase Receptor", pages 895-903, see entire document.
- Molecular and Cellular Biology, Volume 11, No. 1, issued January 1991, D.S. MIDDLEMAS et al., "trkB, a Neural Receptor Protein-Tyrosine Kinase: Evidence for a Full-Length and Two Truncated Receptors", pages 143-153, see entire document.
- Cell, Volume 66, No. 2, issued 26 July 1991, R. KLEIN et al., "The trkB Tyrosine Protein Kinase is a Receptor for Brain-Derived Neurotrophic Factor and Neurotrophin-3", pages 395-403, see entire document.
- Gene, Volume 85, issued 1989, A.F. WILKS et al., "The application of the polymerase chain reaction to cloning members of the protein tyrosine kinse family", pages 67-74, see entire document.
- Science, Volume 241, issued 1 July 1988, S.K. HANKS et al., "The Protein Kinase Family: Conserved Features and Deduced Phylogeny of the Catalytic Domains", pages 42-52, see entire document.

## Description

The present invention relates to the cloning of a novel tyrosine kinase receptor.

The development and maintenance of the vertebrate nervous system depends on specific proteins, termed neurotrophic factors, originally defined by their ability to support the survival of neuronal populations (Snider and Johnson, 1989, Ann. Neurol. 26:489). Neurotrophic factors have also been implicated in processes involving the proliferation and differentiation of neurons (Cattaneo and McKay, 1990, Nature 347: 762-765; Lindsay and Harmar, 1989, Nature 337: 362-364), and they may play additional, thus far unexplored, roles both within as well as outside of the nervous system. Brain-derived neurotrophic factor (BDNF) and neurotrophin-3 (NT-3) have recently been molecularly cloned and shown to be structurally related to the prototypical neuronal survival molecule, nerve growth factor (NGF; Leibrock et al., 1989, Nature 341:149-152; Hohn et al., 1990, Nature 344:339-341; Maisonpierre et al., 1990a, Science 247:1446-1451; Rosenthal et al., 1990, Neuron 4:767-773; Ernfors et al., 1990, Proc. Natl. Acad. Sci. U.S.A. 87:5454-5458; Jones and Reichardt, 1990, Proc. Natl. Acad. Sci. U.S.A. 87:8060-8064). These three related factors (designated "neurotrophins") do not display any structural homology to a fourth neurotrophic factor, ciliary neurotrophic factor (CNTF; Lin et al., 1989, Science 246:1023-1025; Stockli et al., 1989, Nature 342:920-923).

The receptor and signal transduction pathways utilized by NGF have been extensively studied, in large part due to the availability of a pheochromocytoma cell line (PC12) which differentiates in response to NGF (Greene and Tischler, 1976, Proc. Natl. Acad. Sci. U.S.A. 73:2424). These studies have resulted in the cloning of a transmembrane protein (designated "LNGFR" for low-affinity NGF receptor) which binds NGF with relatively low affinity (Chao et al., 1986, Science 232:518-521; Radeke et al., 1987, Nature 325:593-597). In addition to the LNGFR another protein (designated "HNGFR" for high-affinity NGF receptor), which is involved in forming a higher affinity binding site for NGF, is apparently required to initiate NGF-induced signal transduction (Zimmerman et al., 1978, J. Supramol. Struc. 9:351-361; Sutter et al., 1979 in Transmembrane Signalling (N.Y. Alan Liss) pp. 659-667; Bernd and Greene, 1984, J. Bio. Chem. 259:15509-15516; Hempstead et al., 1989, Science 243:373-375). This HNGFR is phosphorylated on tyrosine in response to NGF, and apparently contains intrinsic tyrosine kinase activity (Meakin and Shooter, 1991a, Neuron 6:153-163). Furthermore, the ERK kinases (also known as the MAP2 kinases), early intermediates in tyrosine kinase activated signal cascades, are rapidly activated and phosphorylated on tyrosine in response to NGF. Thus, like many other growth factor responses, NGF signal transduction may be initiated by the activation of a receptor-linked tyrosine kinase.

Recent studies have revealed that the product of the trk proto-oncogene, which resembles a growth factor receptor (i.e., it is a transmembrane protein containing an intracytoplasmic tyrosine kinase domain) for which no ligand had been identified, is rapidly phosphorylated in response to NGF treatment in PC12 cells (Kaplan et al., 1991, Nature 350:156-160; Klein et al., 1991, Cell 65:189-197) and to directly bind NGF with relatively high affinity when expressed in heterologous cells (Klein et al. supra). This finding, together with the restricted neuronal distribution of the trk protein in vivo, suggests that trk may be the component of the HNGFR responsible for initiating NGF signal transduction.

In contrast to the extensive study of NGF receptors and signal transduction pathways, the receptors and signal transduction pathways utilized by the other neurotrophic factors have only recently begun to be explored. However, BDNF appears to bind to the LNGFR with an affinity similar to that of NGF (Rodriguez-Tebar et al., 1990, Neuron 4:487-492). Although both low and high affinity receptors for BDNF exist on neurons responsive to BDNF, the findings that BDNF and NGF act on different neurons and that NGF-responsive neurons do not express high-affinity BDNF receptors suggest that BDNF utilizes a different high affinity receptor than NGF (Rodriguez-Tebar and Barde, 1988, J. Neurosc. 8:3337-3342).

A variety of findings seem to link BDNF and NT-3, while distinguishing both of these neurotrophins from NGF. NT-3 and BDNF (but not NGF) expression displays striking reciprocal relationships during development, with NT-3 being expressed more prominently early and BDNF more prominently late during the development of some of the same brain regions (Maisonpierre et al., 1990, Neuron 5: 501-509). Interestingly, the distribution profiles that BDNF and NT-3 (but not NGF) ultimately achieve in various adult brain regions are quite similar. Id. In peripheral ganglia both BDNF and NT-3 (but not NGF) have their major effects on dorsal root ganglia and nodose ganglia, although NT-3 does seem to have minor effects on sympathetic ganglia (Maisonpierre et al. 1990a, Science 247: 1446-1451). NGF, in contrast, predominantly affects dorsal root ganglia and sympathetic ganglia.

These findings led to the suggestion that BDNF and NT-3 might in some cases act on the same neuronal populations, and that an early effect of NT-3 on these neurons might be replaced by a later effect of BDNF (Maisonpierre et al., 1990b, Neurons 5:501-509). Furthermore, the finding that BDNF and NT-3 (but not NGF) are the most highly conserved growth factors yet described led to the suggestion that both these factors might be interacting with multiple receptors and that their strict conservation was required to maintain the specificity of their interactions with these multiple receptors.

Klein et al. (1989, EMBO J. 8:3701-3709) reported the isolation of trkB, which encodes a new member of the tyrosine protein kinase family of receptors found to be highly related to the human trk protooncogene. At the amino acid level, the products of trk and trkB were found to share 57 percent homology in their extracellular regions, including 9 of the 11 cysteines present in trk. This homology was found to increase to 88 percent within their respective tyrosine kinase catalytic domains. In adult mice, trkB was found to be preferentially expressed in brain tissue, although significant levels of trkB RNAs were also observed in lung, muscle, and ovaries. Further, trkB transcripts were detected in mid and late gestation embryos. In situ hybridization analysis of 14 and 18 day old mouse embryos indicated that trkB transcripts were localized in the central and peripheral nervous systems, including brain, spinal cord, spinal and cranial ganglia, paravertebral trunk of the sympathetic nervous system and various innervation pathways, suggesting that the trkB gene product may be a receptor involved in neurogenesis and early neural development as well as playing a role in the adult nervous system.

In 1990, Klein et al. (Cell 61:647-656) reported that the mouse trkB locus codes for at least two classes of receptor-like molecules, which they designated gp145^{trkB} and gp95^{trkB}. These molecules appear to have identical extracellular and transmembrane domains, but only gp145^{trkB} was found to contain a long cytoplasmic region that included a catalytic protein kinase domain. TrkB transcripts coding for this protein were observed in the cerebral cortex and the pyramidal cell layer of the hippocampus, whereas transcripts encoding gp95^{trkB} were found in the ependymal linings of the cerebral ventricles and in the choroid plexus. Further, Middlemas et al. (1991, Mol. Cell. Biol. 11:143-153) reported the existence of two distinct C-terminally truncated receptors which share the complete extracellular region and transmembrane domain with gp145^{trkB} but which differ from gp145^{trkB} (hitherto referred to simply as trkB) in their short cytoplasmic tails.

The present invention relates to a novel tyrosine kinase receptor referred to herein as Rtk-2.

Accordingly the present invention provides a purified protein comprising an amino acid sequence encoded by the human nucleotide sequence contained in the plasmid pBluescript SK-containing Rtk-2 as deposited with the American Type Culture Collection (ATCC) and assigned accession no. 75052.

The present invention further provides an isolated nucleic acid comprising a nucleotide sequence which encodes the protein of the invention.

The present invention also has diagnostic and therapeutic utilities.

### ABBREVIATIONS

- BDNF: brain derived neurotrophic factor
- BSA: bovine serum albumin
- CNTF: ciliary neurotrophic factor
- DSS: Disuccinimidyl suberate
- HNGFR: high affinity nerve growth factor receptor
- LNGFR: low affinity nerve growth factor receptor
- NGF: nerve growth factor
- NT-3: neurotrophin-3

Using a method for identifying receptor molecules, an orphan tyrosine kinase receptor-like molecule, referred to herein as Rtk-2, which is homologous to trk receptor and the insulin receptor family was cloned.

The method comprised (i)amplifying tyrosine kinase encoding nucleic acid sequences by polymerase chain reaction using a collection of cDNA molecules as template and using oligonuleotide primers that correspond to regions of known tyrosine kinase molecules, said regions being associated with tyrosine kinase activity; and (ii) cloning the amplified nucleic acid into a plasmid.

DNA amplified and cloned by this method was then sequenced using standard techniques. The resulting sequences were then compared to the sequences of known tyrosine kinase molecules in order to identify clones of particular interest.

Accordingly, the present invention provides an isolated nucleic acid comprising the human nucleotide sequence contained in the plasmid pBluescript SK-containing Rtk-2 as deposited with the American Type Culture Collection (ATCC) and assigned accession no. 75052. In one embodiment of the present invention portions of the nucleic acid comprising at least ten nucleic acid residues are also provided.

The present invention further provides a purified protein comprising an amino acid sequence encoded by the human nucleotide sequence contained in the plasmid pBluescript SK-containing Rtk-2 as deposited with the American Type Culture Collection (ATCC) and assigned accession no. 75052. In one embodiment of the present invention portions of the protein comprising at least six amino acid molecules, or functionally equivalent molecules are also provided.

Functionally equivalent molecules include those in which amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolor (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

Also included within the scope of the invention are proteins or fragments or derivatives thereof which are differentially modified during or after translation, e.g. by glycosylation, proteolytic cleavage, linkage to an antibody molecule or other cellular ligand, etc.

The present invention also provides a recombinant vector comprising the nucleic acid of the invention.

The present invention further provides for cells and microorganisms that carry the recombinant nucleic acid molecules of the invention. In particular embodiments, the cell carrying the recombinant nucleic acid is a fibroblast. In other embodiments, the cell is a bacterium.

The present invention also provides a method for producing a Rtk-2 protein comprising growing a recombinant cell containing the vector of the invention such that the Rtk-2 protein encoded by the vector is expressed by the cell, and recovering the expressed Rtk-2 protein. Also provided by the present invention is the purified product of the method.

Amplified nucleic acid fragments isolated using the cloning method as described above may be used to identify full-length cDNA clones. In preferred embodiments of the invention, an amplified DNA fragment of interest is used to identify a tissue or cell line that expresses relatively abundant levels of a corresponding mRNA, for example, using Northern blot or dot-blot analysis. Such a tissue or cell line may then be used to generate a cDNA library which may serve as a superior source of a full length cDNA which comprises the sequence and the amplified fragment.

### EXAMPLE: CLONING OF ORPHAN TRK-LIKE RECEPTOR MOLECULES

### MATERIALS AND METHODS

PCR-mediated cloning was performed using cDNA from the human neuroblastoma cell line SY5Y as template, and oligonucleotide primers shown in Table I, infra.

### RESULTS AND DISCUSSION

The goal of this work was to identify, by PCR, DNA sequences closely related to trk and trkB genes. Thus, oligodeoxynucleotide primers were designed which correspond to protein regions strongly conserved in all protein kinases, but which at the same time would strongly bias the amplification reaction towards trk-related sequences. For example, trk-10r primer corresponds to the -PIRWMPPE- in which trk and trkB are identical,but even their closest known relatives, insulin and insulin-like growth factor receptors have two amino acid substitutions.

Using cDNA from human neuroblastoma cell line SY5Y as a template, polymerase chain reactions (PCRs) with various combinations of primers were performed. The DNA products were digested with restriction enzymes which cut trk and trkB sequences, and the resistant material was re-amplified and cloned. Individual clones were then sequenced. Amino acid sequences deduced from these DNA sequences revealed the presence of fragments of four novel potential receptors in the pool. These fragments were preferred to as Rtk-2, Rtk-3, Rtk-4 and Rtk-5.

The following oligodeoxynucleotide primers were used in preparation of the PCR fragments (the restriction site "tail" used for cloning is shown in italics, and the degeneracy at particular position is indicated by the letters in parentheses):

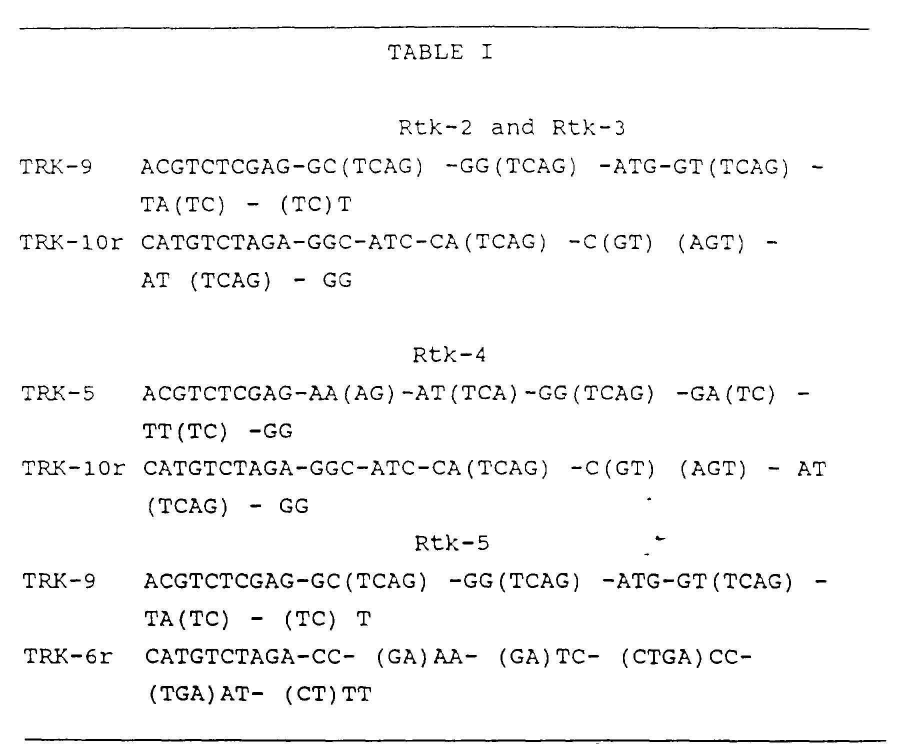

Rtk-2 and Rtk-3 sequences were of particular interest. When the deduced amino acid sequences were used to search GenBank, Release 67.0 (03/91) and EMBL (Modified) Release 26.0 (02/91) Databases with TFASTA, the highest homology scores were obtained for trkB and trk sequences. Rtk-2 and Rtk-3 contain the YxxDYY sequence characteristic for trk/insulin-R subfamily. Both sequences have an L for F substitution in the DFG motif which is very strongly conserved in all kinases.

When the Rtk-2 and Rtk-3 probes were hybridized to Northern blots, no signal was obtained with SY5Y RNA. Rtk-2 hybridized to a 6-7 kb band in RNAs from other lines, in particular from CHP100, SKES, and LAN5 cells. Rtk-3 hybridized to a 5 kb band in RNAs from SKN-SH and LAN5 cells. Screening of 1.5 x 10⁶ clones from SY5Y cDNA library in lambda ZPA II with Rtk-2 probe yielded 5 positive clones, with inserts in the range 1.4-3 kb.

Sequence data for Rtk-2, indicates nucleotides that appear to code for a transmembrane domain, nucleotides that appear to contain a tyrosine kinase domain; and nucleotides that code for a continuous open reading frame comprising a ligand binding domain. A 180 amino acid stretch follows the tyrosine kinase domain, in contrast to trks, which terminate shortly following the tyrosine kinase domain.

The partial amino acid sequence of Rtk-3, which also shows the presence of this stretch beyond the tyrosine kinase domain and shows strong homology to Rtk-2.

Alignment of the Rtk-3 sequence to the sequences for Rtk-2, trks, IGFR, and Insulin receptor indicates that Rtk-2 and Rtk-3 share the greatest homology, suggesting they are members of a novel subfamily of tyrosine kinase receptors.

### DEPOSIT OF MICROORGANISMS

The following microorganisms have been deposited with the American Type Culture Collection, 12301 Parklawn Drive, Rockville, Maryland 20852.

| | ACCESSION NUMBER |
|---|---|
| pBluescript SK-containing Rtk-2 | 75052 |
| pBluescript SK-containing Rtk-3 | 75053 |

## Claims (Claims for the following Contracting State(s): AT, BE, CH, LI, DE, DK, FR, GB, IT, LU, MC, NL, SE)

1. A purified protein comprising an amino acid sequence encoded by the human nucleotide sequence contained in the plasmid pBluescript SK-containing Rtk-2 as deposited with the American Type Culture Collection (ATCC) and assigned accession no. 75052.

2. An isolated nucleic acid comprising the human nucleotide sequence contained in the plasmid pBluescript SK-containing Rtk-2 as deposited with the American Type Culture Collection (ATCC) and assigned accession no. 75052.

3. An isolated nucleic acid comprising a nucleotide sequence which encodes the protein of claim 1.

4. A recombinant vector comprising the nucleic acid of claim 2 or 3.

5. A recombinant host cell containing the vector of claim 4.

6. A method for producing a Rtk-2 protein comprising growing a recombinant cell containing the vector of claim 4 such that the Rtk-2 protein encoded by said vector is expressed by the cell, and recovering the expressed Rtk-2 protein.

7. The purified product of the method of claim 6.

## Claims (Claims for the following Contracting State(s): ES, GR)

1. A method for producing a Rtk-2 protein comprising growing a recombinant cell containing a vector, which vector contains a nucleic acid comprising the human nucleotide sequence contained in the plasmid pBluescript SK-containing Rtk-2 as deposited with the American Type Culture Collection (ATCC) and assigned accession no. 75052, such that the Rtk-2 protein encoded by said vector is expressed by the cell, and recovering the expressed Rtk-2 protein.

2. A method according to claim 1, further comprising purifying the recovered expressed Rtk-2 protein.

3. The purified product of the method of claim 1 or 2.

4. A purified protein comprising an amino acid sequence encoded by the human nucleotide sequence contained in the pBluescript SK-containing Rtk-2 as deposited with the American Type Culture Collection (ATCC) and assigned accession no. 75052.

5. An isolated nucleic acid comprising the human nucleotide sequence contained in the plasmid pBluescript SK-containing Rtk-2 as deposited with the American Type Culture Collection (ATCC) and assigned accession no. 75052.

6. An isolated nucleic acid comprising a nucleotide sequence which encodes the protein of claim 4.

7. A recombinant vector comprising the nucleic acid of claim 5 or 6.

8. A recombinant host cell containing the vector of claim 7.

## Patentansprüche (Patentansprüche für folgende(n) Vertragsstaat(en): AT, BE, CH, LI, DE, DK, FR, GB, IT, LU, MC, NL, SE)

1. Gereinigtes Protein, umfassend eine Aminosäuresequenz, die von der menschlichen Nucleotidsequenz codiert wird, die in dem Plasmid pBluescript SK, enthaltend Rtk-2, enthalten ist, hinterlegt bei der American Type Culture Collection (ATTC) unter der Hinterlegungsnummer 75052.

2. Isolierte Nucleinsäure, umfassend die menschliche Nucleotidsequenz, die in dem Plasmid pBluescript SK, enthaltend Rtk-2, enthalten ist, hinterlegt bei der American Type Culture Collection (ATTC) unter der Hinterlegungsnummer 75052.

3. Isolierte Nucleinsäure, umfassend eine Nucleotidsequenz, die das Protein nach Anspruch 1 codiert.

4. Rekombinanter Vektor, umfassend die Nucleinsäure nach Anspruch 2 oder 3.

5. Rekombinante Wirtszelle, enthaltend den Vektor nach Anspruch 4.

6. Verfahren zur Produktion eines Rtk-2-Proteins, umfassend die Züchtung einer rekombinanten Zelle, die den Vektor nach Anspruch 4 enthält, so dass das Rtk-2-Protein, das von dem Vektor codiert wird, in der Zelle exprimiert wird, und Gewinnung des exprimierten Rtk-2-Proteins.

7. Gereinigtes Produkt des Verfahrens nach Anspruch 6.

## Patentansprüche (Patentansprüche für folgende(n) Vertragsstaat(en): ES, GR)

1. Verfahren zur Produktion eines Rtk-2-Proteins, umfassend die Züchtung einer rekombinanten Zelle, die einen Vektor enthält, wobei der Vektor eine Nucleinsäure umfassend die menschliche Nucleotidsequenz, die in dem Plasmid pBluescript SK, enthaltend Rtk-2, enthalten ist, hinterlegt bei der American Type Culture Collection (ATTC) unter der Hinterlegungsnummer 75052, enthält, so dass das Rtk-2-Protein, das von dem Vektor codiert wird, in der Zelle exprimiert wird, und Gewinnung des exprimierten Rtk-2-Proteins.

2. Verfahrer nach Anspruch 1, weiterhin umfassend die Reinigung des gewonnenen exprimierten Rtk-2-Proteins.

3. Gereinigtes Produkt des Verfahrens nach Anspruch 1 oder 2.

4. Gereinigtes Protein, umlassend eine Aminosäuresequenz, die von der menschlichen Nucleotidsequenz codiert wird, die in dem Plasmid pBluescript SK, enthaltend Rtk-2, enthalter ist, hinterlegt bei der American Type Culture Collection (ATTC) unter der Hinterlegungsnummer 75052.

5. Isolierte Nucleinsäure, umfassend die menschliche Nucleotidsequenz, die in dem Plasmid pBluescript SK enthaltend Rtk-2, enthalten ist, hinterlegt bei der American Type Culture Collection (ATTC) unter der Hinterlegungsnummer 75052.

6. Isolierte Nucleinsäure, umfassend eine Nucleotidsequenz, die das Protein nach Anspruch 4 codiert.

7. Rekombinanter Vektor, umfassend die Nucleinsäure nach Anspruch 5 oder 6.

8. Rekombinante Wirtszelle enthaltend den Vektor nach Anspruch 7.

## Revendications (Revendications pour l'(les) Etat(s) contractant(s) suivant(s): AT, BE, CH, LI, DE, DK, FR, GB, IT, MC, NL, SE)

1. Protéine purifiée, comprenant une séquence d'aminoacides codée par la séquence nucléotidique humaine contenue dans le plasmide pBluescript SK contenant Rtk-2, déposé à l'American Type Culture Collection (ATCC) et ayant reçu le n° de dépôt 75052.

2. Acide nucléique isolé, comprenant la séquence nucléotidique humaine contenue dans le plasmide pBluescript SK contenant Rtk-2, déposé à l'American Type Culture Collection (ATCC) et ayant reçu le n° de dépôt 75052.

3. Acide nucléique isolé, comprenant une séquence nucléotidique qui code pour la protéine de la revendication 1.

4. Vecteur recombinant, comprenant l'acide nucléique de la revendication 2 ou 3.

5. Cellule hôte recombinante, contenant le vecteur de la revendication 4.

6. Procédé de production d'une protéine Rtk-2, comprenant la culture d'une cellule recombinante contenant le vecteur de la revendication 4, de sorte que la protéine Rtk-2 codée par ledit vecteur est exprimée par la cellule, et la récupération de la protéine Rtk-2 exprimée.

7. Le produit purifié du procédé de la revendication 6.

## Revendications (Revendications pour l'(les) Etat(s) contractant(s) suivant(s): ES, GR)

1. Procédé de production d'une protéine Rtk-2, comprenant la culture d'une cellule recombinante contenant un vecteur, lequel vecteur contient un acide nucléique, comprenant la séquence nucléotidique humaine contenue dans le plasmide pBluescript SK contenant Rtk-2, déposé à l'American Type Culture Collection (ATCC) et ayant reçu le n° de dépôt 75052, de sorte que la protéine Rtk-2 codée par ledit vecteur est exprimée par la cellule, et la récupération de la protéine Rtk-2 exprimée.

2. Procédé selon la revendication 1, comprenant en outre la purification de la protéine Rtk-2 exprimée et récupérée.

3. Le produit purifié du procédé de la revendication 1 ou 2.

4. Protéine purifiée, comprenant une séquence d'aminoacides codée par la séquence nucléotidique humaine contenue dans le plasmide pBluescript SK contenant Rtk-2, déposé à l'American Type Culture Collection (ATCC) et ayant reçu le n° de dépôt 75052.

5. Acide nucléique isolé, comprenant la séquence nucléotidique humaine contenue dans le plasmide pBluescript SK contenant Rtk-2, déposé à l'American Type Culture Collection (ATCC) et ayant reçu le n° de dépôt 75052.

6. Acide nucléique Isolé, comprenant une séquence nucléotidique qui code pour la protéine de la revendication 4.

7. Vecteur recombinant, comprenant l'acide nucléique de la revendication 5 ou 6.

8. Cellule hôte recombinante, contenant le vecteur de la revendication 7.
